# EUROPÄISCHE PATENTSCHRIFT

(11) **EP 3 488 281 B1**
(45) Veröffentlichungstag und Bekanntmachung des Hinweises auf die Patenterteilung: **26.07.2023**
(21) Anmeldenummer: 17784567.4
(22) Anmeldetag: 27.09.2017
(51) Int. Cl.: G02B 21/00, A61B 90/20, G06T 7/00

(54) **ERMITTELN EINES GEWEBETYPS EINES GEWEBES EINES TIERISCHEN ODER MENSCHLICHEN INDIVIDUUMS**
DETERMINING A TISSUE TYPE OF A TISSUE OF AN ANIMAL OR HUMAN INDIVIDUAL
DÉTERMINATION D'UN TYPE DE TISSU ANIMAL OU HUMAIN

(30) Priorität: 27.09.2016 DE 102016218520
(43) Veröffentlichungstag der Anmeldung: 29.05.2019
(73) Patentinhaber: Siemens Healthcare GmbH, 91052 Erlangen (DE)
(72) Erfinder: ENGEL, Thomas, 73432 Aalen (DE); GIGLER, Alexander Michael, 86836 Untermeitingen (DE); OTTE, Clemens, 81739 München (DE); PASTUSIAK, Remigiusz, 81549 München (DE); PAUST, Tobias, 89340 Leipheim (DE); SIMON, Elfriede, 80639 München (DE); STÜTZ, Evamaria, 81827 München (DE); VOGL, Stefanie, 94357 Konzell (DE)
(86) Internationale Anmeldenummer: PCT/EP2017/074488
(87) Internationale Veröffentlichungsnummer: WO 2018/060243

(56) Entgegenhaltungen:
- WO-A1-2015/154187
- US-A- 5 999 844
- US-A1- 2005 010 102
- US-A1- 2015 011 893
- Anonymous: "Online machine learning - Wikipedia", , 11 September 2016 (2016-09-11), pages 1-11, XP093004289, Retrieved from the Internet: URL:https://en.wikipedia.org/w/index.php?t itle=Online_machine_learning&oldid=7389215 30 [retrieved on 2022-12-02]
- Anonymous: "Online machine learning - Wikipedia, the free encyclopedia", , 4 August 2014 (2014-08-04), XP055132995, Retrieved from the Internet: URL:http://en.wikipedia.org/wiki/Online_ma chine_learning [retrieved on 2014-08-04]

## Beschreibung

Die Erfindung betrifft ein Verfahren zum Ermitteln eines Gewebetyps eines Gewebes eines tierischen oder menschlichen Individuums, bei dem durch eine Gewebeprobe des Gewebes abgegebene elektromagnetische Strahlung mittels eines Strahlungssensors erfasst wird, wobei der Strahlungssensor abhängig von der erfassten elektromagnetischen Strahlung ein Sensorsignal bereitstellt, und das Sensorsignal mittels einer Auswerteeinheit ausgewertet wird, um den Gewebetyp zu ermitteln.

Verfahren der gattungsgemäßen Art, Verwendungen solcher Verfahren zur Unterstützung eines Operateurs bei einem Eingriff in den Körper des Individuums, Rechnerprogrammprodukte sowie auch gattungsgemäße Vorrichtungen sind im Stand der Technik umfänglich bekannt, sodass es eines gesonderten druckschriftlichen Nachweises hierfür nicht bedarf. Insbesondere bei einem chirurgischen Eingriff in den Körper des Individuums durch den Operateur, der in der Regel ein Arzt ist, ist der optische Eindruck des Operateurs, seine Expertise und seine Erfahrung für eine Entscheidung von größter Bedeutung, wie der chirurgische Eingriff durchgeführt werden soll. Dabei ist es wichtig, dass der Operateur erkennt, welche Gewebetypen an der Stelle, an der der chirurgische Eingriff vorgenommen wird, angeordnet sind, in welchem Zustand das jeweilige Gewebe ist sowie auch die Feststellung, ob das vorliegende gewebeentzündliche, nekrotische, bösartige oder verletzungsbedingte Veränderungen aufweist. Derartige Gewebe werden als "auffälliges Gewebe" bezeichnet. Falls auffälliges Gewebe vorliegen sollte, liegt es in der Entscheidung des Operateurs, auf Basis seines optischen Eindrucks zu entscheiden, wieviel von gegebenenfalls auffälligem Gewebe entfernt werden muss, wo eine Grenze zwischen einem normalen, gesunden Gewebe zum auffälligen Gewebe ist, und gegebenenfalls wieviel vom normalen beziehungsweise gesunden Gewebe entfernt werden muss, um beste Voraussetzungen für eine schnelle Genesung des Individuums beziehungsweise des Patienten schaffen zu können.

Maßgeblich beeinflusst wird die Sicht und die Entscheidung des Operateurs bezüglich der Entfernung von Gewebe durch einen Grad und eine makroskopisch sichtbare Auffälligkeit des auffälligen Gewebes gegenüber dem gesunden beziehungsweise normalen Gewebe und die Einsehbarkeit sowie auch die Zugänglichkeit des Operationsfeldes am Individuum beziehungsweise dem Bereich, in dem der chirurgische Eingriff vorgenommen wird.

Bei derartigen chirurgischen Eingriffen ist es für den Operateur oftmals schwierig, eine makroskopische Struktur von auffälligem Gewebe, beispielsweise einem Tumor oder dergleichen, von einem normalen beziehungsweise gesunden Gewebe abgrenzen zu können. Besonders schwierig erweist es sich für den Operateur, lediglich anhand seiner visuellen Eindrücke zu entscheiden, wo eine Grenze zwischen normalen beziehungsweise gesunden Gewebe gegenüber einem auffälligen Gewebe, insbesondere einem bösartigen, malignen Gewebe verläuft, und ob zum Beispiel bei einer Sektion eines Tumors tatsächlich auch sämtliche Randbereiche des Tumors vollständig entfernt werden.

Wird beispielsweise ein Tumor resektiert, ist es mittlerweile üblich, das entfernte Gewebe direkt in ein pathologisches Labor zu übersenden, wo es unmittelbar histologisch untersucht wird, damit festgestellt werden kann, ob tatsächlich Grenzen des entfernten Tumors in dem entfernten Gewebe enthalten sind und somit der komplette Tumor entfernt werden konnte. Wird im Rahmen der Untersuchung festgestellt, dass der Tumor nicht vollständig entfernt werden konnte, wird der Operateur entsprechend unmittelbar informiert, sodass der Operateur in geeigneter Weise weiteres Gewebe entfernen kann, um auch bislang noch nicht entfernte Tumorgrenzen erfassen zu können. Für die benötigte Zeit der histologischen Untersuchung wird der chirurgische Eingriff beziehungsweise die Operation unterbrochen, und das Individuum wird währenddessen weiterhin narkotisiert gehalten, bis ein endgültiges Ergebnis der histologischen Untersuchung vorliegt.

Besonders schwierig erweist sich die optische, makroskopische Evaluation des Gewebes, wenn der Bereich des chirurgischen Eingriffs beziehungsweise der Operationsbereich nur schwer einsehbar ist, beispielsweise wenn der Tumor direkt bei oder in überlebenswichtigen Organen oder Geweben positioniert ist, wie beispielsweise dem Gehirn oder dergleichen. Weiter erschwerend kann es sein, wenn der Tumor dann auch noch durch Knochen, wie zum Beispiel einer Schädeldecke, verdeckt ist.

Bisher ist es üblich, dass sich der Operateur weitgehend auf seine Erfahrung stützt, das optische Bild im Sichtfeld zu interpretieren und dadurch zu erkennen, welcher Gewebetyp vorliegt, insbesondere ob es sich bei dem vorliegenden Gewebe um normales beziehungsweise gesundes Gewebe oder auffälliges Gewebe, insbesondere Tumorgewebe, handelt. Insbesondere nutzt er seine Erfahrung, um eine Grenze zwischen normalem beziehungsweise gesundem Gewebe und auffälligem Gewebe, welches entfernt werden soll, zu bestimmen.

Mittlerweile ist es auch möglich, den optischen Eindruck, den der Operateur gewonnen hat, ergänzend durch haptische Eindrücke des jeweiligen Gewebes zu unterstützen. Dabei wird die Erkenntnis genutzt, dass unterschiedliche Gewebetypen, insbesondere auch Unterschiede zwischen normalem beziehungsweise gesundem Gewebe in Bezug auf auffälliges Gewebe, beispielsweise Tumorgewebe, zum Teil Unterschiede bezüglich einer Härte oder Festigkeit aufweisen können.

Bei einer Tumorresektion erfolgt während der Operation beziehungsweise dem chirurgischen Eingriff zum Beispiel eine histologische Untersuchung beziehungsweise Beurteilung des entnommenen Gewebes durch sogenannte Schnellschnitte, aus denen der Pathologe ermitteln kann, ob tatsächlich Tumorgrenzen erfasst wurden, insbesondere vollständig erfasst wurden, oder ob noch Tumorgewebe beim Individuum verblieben ist. Während der hierfür erforderlichen Zeit wird die Operation beziehungsweise der chirurgische Eingriff unterbrochen, und das Individuum wird weiterhin unter Narkose gehalten.

Für die Durchführung des chirurgischen Eingriffs beziehungsweise der Operation muss der Operationsbereich für den Operateur natürlich möglichst gut einsehbar sein, das heißt, eine Wunde, die durch den chirurgischen Eingriff beziehungsweise die Operation entsteht, kann relativ groß sein. Dadurch kann sich eine hohe Belastung für das Individuum, beispielsweise durch eine lange und hohe Anästhesie, einen großen Blutverlust und oder dergleichen, ergeben, wodurch ein späterer Heilungsprozess des Individuums beeinträchtigt sein kann.

Eine Möglichkeit, die durch eine Operation beziehungsweise durch einen chirurgischen Eingriff verursachte Wunde möglichst klein zu halten, kann durch eine endoskopische Operation erreicht werden, die eine sogenannte minimal-invasive Operation darstellt. Diese Art der Operation wird in einigen Bereichen mittlerweile standardmäßig eingesetzt, zum Beispiel bei neurochirurgischen Eingriffen, wie zum Beispiel bei einer Operation an einer Bandscheibe, Behandlungen des eines Hydrozephalus, bei einem Eingriff im Bauchraum, zum Beispiel bei einer Operation am Blinddarm, der Galle, eines Leistenbruchs, oder auch bei Eingriffen an Gelenken, sogenannte Gelenksspiegelungen, und/oder dergleichen. Bei einer endoskopischen Operation werden die chirurgischen und/oder optischen Instrumente durch dünne Metallkanäle in den Körper des Individuums eingeführt. Durch eine Kamera, insbesondere im Bereich einer Spitze des Endoskops, kann ein Bild aus dem Körper des Individuums visuell bereitgestellt werden, welches zum Beispiel mittels einer Anzeigeeinrichtung wie einem Monitor oder dergleichen dargestellt werden kann.

Ein weiteres Hilfsmittel für den Operateur, den Bereich der Operation beziehungsweise des chirurgischen Eingriffs optisch verbessert einsehen zu können, ist ein Operationsmikroskop.

Das Operationsmikroskop wird in der Regel über dem Bereich der Operation beziehungsweise des chirurgischen Eingriffs angeordnet und kann diesen Bereich in geeigneter Weise vergrößert darstellen, beispielsweise mit einer Vergrößerung von bis zu etwa 40-fach oder dergleichen. Die Einstellung der Vergrößerung kann zum Beispiel an einem Objektiv und/oder auch digital durch Darstellung auf einem Monitor oder dergleichen erfolgen. Das Operationsmikroskop kann den Operateur dadurch bei einer genaueren Spezifizierung des Gewebes hinsichtlich des Gewebetyps unterstützen.

Eine weitere Verbesserung kann dadurch erreicht werden, dass als Operationsmikroskop ein Fluoreszenzmikroskop eingesetzt wird. Hierdurch können Eigenschaften von Fluoreszenzfarbstoffen genutzt werden, die dem Individuum entweder zuvor verabreicht oder topisch während der Operation beziehungsweise des chirurgischen Eingriffs auf die zu untersuchende Stelle appliziert werden. Durch die Verwendung eines geeigneten Fluoreszenzfarbstoffs, wie zum Beispiel 5-Aminolävulinsäure, 5-ALA, kann interoperativ eine spezifische Einfärbung zum Beispiel von Tumorgewebe erreicht werden, welche dann mit dem Fluoreszenzmikroskop für den Operateur sichtbar gemacht werden kann. Ein derartiges Verfahren offenbart zum Beispiel Q. T. Nguyen et al. in Fluorescence-guided Surgery with Live Molecular Navigation - a neu Cutting Edge in HHS Public Access in PMC 2015 Mai 11.

Bei dem Einsatz von 5-ALA zeigen sich Tumorzellen unter dem Fluoreszenzmikroskop als rote Zellen. Nicht-Tumorzellen akkumulieren diesen Stoff nicht und sind daher nicht als rote Zellen sichtbar. Fluoreszenzfarbstoffe haben den Vorteil, dass Unterschiede, die der Operateur ohne solche Farbstoffe nicht erkennen könnte, zum Beispiel eine Unterscheidung von Tumorgewebe und Nicht-Tumorgewebe oder dergleichen, klar sichtbar machen kann. Die ansonsten nicht oder nur schlecht sichtbaren Unterschiede können also besser sichtbar gemacht werden. Dadurch kann der Operateur bei Erkennen der Grenze zwischen normalen beziehungsweise gesunden Gewebe und Tumorgewebe unterstützt werden, sodass Tumorgewebe mit einer grö-βeren Sicherheit und effizienter entfernt werden kann.

Bei Fluoreszenzfarbstoffen ist jedoch anzumerken, dass die Aufnahme dieser Stoffe das Individuum beziehungsweise den Patienten belasten kann. Darüber hinaus gibt es nur sehr wenige, insbesondere für den Menschen, zugelassene Farbstoffe, weil diese größtenteils Nebenwirkungen verursachen beziehungsweise verursachen können. Ein solcher Stoff ist beispielsweise in der Forschung am Tiermodell Acriflavin, das als bildgebungsunterstützender Fluoreszenzfarbstoff genutzt wird. Da dieser Fluoreszenzfarbstoff mit der DNA einer Zelle interagieren kann, kann er somit mutagen wirken und ist deshalb für die Anwendung beim Menschen nicht zugelassen.

Darüber hinaus sind Vorrichtungen bekannt, die die Vorteile der endoskopischen Operation und des Operationsmikroskops kombinieren können, so zum Beispiel durch das Gerät Cellvisio der Firma Mauna Kea. Hier können endoskopischen in-vivo-Fluoreszenzfärbungen von zu untersuchenden Geweben erfasst werden.

Die US 2015/0011893 A1 offenbart eine Evaluation von Hautläsionen durch Raman-Spektroskopie.

Ferner offenbart die US 2005/0010102 A1 eine Vorrichtung zum Charakterisieren von pigmentierten Hautläsionen.

Die US 5 999 844 A offenbart ein Verfahren zur Bildgebung von Gewebe mittels Autofluoreszenz.

Die WO 2015/154187 A1 offenbart ein Verfahren zur Klassifizierung eines Gewebes hinsichtlich Krebs mithilfe der Raman-Spektroskopie.

Auch wenn sich der Stand der Technik bisher bewährt hat, besteht dennoch weiterer Verbesserungsbedarf. Gerade bei der Ermittlung von Gewebetypen sind nach wie vor, insbesondere abhängig vom jeweiligen Einzelfall, große Unsicherheiten zu verzeichnen. Es besteht daher ein Bedarf, den Gewebetyp zuverlässiger ermitteln zu können.

Es ist deshalb die Aufgabe der Erfindung, ein verbessertes Ermitteln des Gewebetyps zu ermöglichen.

Als Lösung wird mit der Erfindung ein Verfahren gemäß dem unabhängigen Anspruch vorgeschlagen.

Vorteilhafte Weiterbildungen ergeben sich durch Merkmale der abhängigen Ansprüche.

Bezüglich eines gattungsgemäßen Verfahrens wird vorgeschlagen, dass die Auswerteeinheit eine selbstlernende Auswerteeinheit ist, die initial anhand von Trainingsdatensätzen auf Basis von wenigstens einem Modell trainiert wird, dass auf einem Verfahren zum maschinellen Lernen basiert, wobei das Trainieren der Auswerteeinheit mittels solcher Trainingsdatensätze erfolgt, die ein jeweiliges Trainingssensorsignal mit einem jeweils zugeordneten Trainingsgewebetyp umfassen, wobei die Trainingsdatensätze die Daten von einer Mehrzahl von unterschiedlichen Individuen umfassen, die in einer geeigneten Datenbank gespeichert sind, und wobei die Auswerteeinheit vor einem Ermitteln des Gewebetyps der Gewebeprobe des Individuums mittels wenigstens eines weiteren bekannten Datensatzes des gleichen Individuums zur Anpassung an das tierische oder menschliche Individuum trainiert wird.

Für ein zur Ausführung der Erfindung geeignetes Rechnerprogrammprodukt, das selbst nicht Teil der Erfindung ist, wird insbesondere vorgeschlagen, dass dieses ein Rechnerprogramm für eine Rechnereinheit einer Auswerteeinheit mit einem Strahlungssensor zum Erfassen von durch eine Gewebeprobe des Gewebes abgegebener elektromagnetischer Strahlung, wobei der Strahlungssensor abhängig von der erfassten elektromagnetischen Strahlung ein Sensorsignal bereitstellt, und einer Auswerteeinheit zum Auswerten des Sensorsignals, um den Gewebetyp zu ermitteln und auszugeben, wobei die Auswerteeinheit eine selbstlernende Auswerteeinheit ist, die ausgebildet ist, initial anhand von Trainingsdatensätzen auf Basis von wenigstens einem Modell trainiert zu werden, das auf einem Verfahren zum maschinellen Lernen basiert, wobei die Auswerteeinheit ausgebildet ist, nach einem Ermitteln des Gewebetyps der Gewebeprobe unter Berücksichtigung von einem diesem Auswerten zugeordneten Datensatz erneut trainiert zu werden, umfasst, wobei das Rechnerprogramm Programmkodeabschnitte eines Programms zum Ausführen der Schritte gemäß dem Verfahren der Erfindung aufweist, wenn das Rechnerprogramm durch die Rechnereinheit ausgeführt wird, sodass die Auswerteeinheit durch Auswerten eines von einem elektromagnetische Strahlung einer Gewebeprobe erfassenden Strahlungssensors bereitgestellten Sensorsignals einen Gewebetyp der Gewebeprobe ermittelt.

Vorrichtungsseitig wird für eine gattungsgemäße Vorrichtung insbesondere vorgeschlagen, dass die Auswerteeinheit eine selbstlernende Auswerteeinheit ist, die ausgebildet ist, initial anhand von Trainingsdatensätzen auf Basis von wenigstens einem Modell trainiert zu werden, das auf einem Verfahren zum maschinellen Lernen basiert, wobei das Trainieren der Auswerteeinheit mittels solcher Trainingsdatensätze erfolgt, die ein jeweiliges Trainingssensorsignal mit einem jeweils zugeordneten Trainingsgewebetyp umfassen.

Die Erfindung nutzt die Möglichkeit, den Operateur bei der Beurteilung und der Erkennung unterschiedlicher Gewebetypen beziehungsweise bei der Unterscheidung zwischen einem normalen beziehungsweise gesunden beziehungsweise gutartigen Gewebe und auffälligem Gewebe, insbesondere Tumorgewebe, intraoperativ durch technische Hilfsmittel zu unterstützen. Dies kann durch Einsatz von Spektroskopie, insbesondere Infrarot-Spektroskopie, Ultraviolett-Spektroskopie, Kombinationen hiervon und/oder dergleichen erfolgen.

Insbesondere die Infrarot-Spektroskopie ermöglicht es, die Unterscheidung von Gewebetypen oder auch von Stoffen aufgrund ihrer molekularen Zusammensetzung zu unterstützen. Dabei wird die Erkenntnis genutzt, dass funktionelle Gruppen von Molekülen durch Anregung von Licht im infrarot-spektralen Bereich unterschiedlich reagieren, wodurch sich bei einer spektralen Auswertung charakteristische Unterschiede ergeben. Entsprechend können somit gewebespezifisch beziehungsweise stoffspezifisch unterschiedliche charakteristische Spektren gewonnen werden. Im Idealfall ist dabei jedes individuelle Spektrum genau einem einzigen Gewebetyp zuordbar, wodurch eine einfache Entscheidung zwischen gesundem beziehungsweise normalem Gewebe und auffälligem Gewebe, insbesondere Tumorgewebe, erreicht werden kann. Bezüglich der Infrarot-Spektroskopie wird ergänzend insbesondere auf Wangdong Ni et al. Non-Linear Calibration Models for Near Infrared Spectroscopy, Analytica Chimica Acta 813 (2014) 1-14 verwiesen.

Als problematisch erweist sich dabei jedoch, dass die molekulare Zusammensetzung beziehungsweise der molekulare Aufbau von normalen beziehungsweise gesunden Gewebe und aus einem solchen Gewebe entstandenen auffälligem Gewebe, insbesondere Tumorgewebe, sehr ähnlich ist, sodass sich zwar bezüglich der ermittelten Spektren beziehungsweise Infrarotspektren Unterschiede ergeben, die jedoch nicht immer auf einzelne bekannte molekulare Unterschiede zurückgeführt werden können, die man als "intrinsischen Marker" für einen bestimmten Gewebetyp nutzen könnte. Ein derartiger Unterschied auf molekularer Ebene, der beispielsweise gutartiges Gewebe von Tumorgewebe unterscheiden könnte, und gleichzeitig mittels Infrarot-Spektroskopie nachweisbar wäre, ist bislang noch nicht bekannt.

Neben verschiedenen Gewebetypen und/oder deren Konditionen von Geweben können aber auch individuelle Unterschiede vorliegen, die zu Abweichungen im Spektrum, insbesondere im Infrarotspektrum, führen können. So kann zum Beispiel ein Infrarotspektrum einer Leber eines ersten Individuums im Vergleich zu einem Infrarotspektrum der Leber eines zweiten Individuums unterschiedlich sein. Es braucht also nicht identisch zu sein, sondern es können individuumindividuelle Abweichungen auftreten. Darüber hinaus können natürlich auch Bedingungen bezüglich der Erfassung der von der Gewebeprobe abgegebenen elektromagnetischen Strahlung, insbesondere der Infrarotstrahlung, zu spektralen Abweichungen führen, so zum Beispiel ein Einfallswinkel der elektromagnetischen Strahlung, mit der die Gewebeprobe beaufschlagt wird, insbesondere ein Einfallswinkel von Licht, vorzugsweise Infrarotlicht.

Um den Operateur insbesondere bei Nutzung der Spektroskopie, beispielsweise der Infrarot-Spektroskopie zu unterstützen, wird deshalb mit der Erfindung eine Auswertemethode vorgeschlagen, die mittels einer Auswerteeinheit realisiert werden kann und die zulässt, ein Sensorsignal, welches mittels des Strahlungssensors bereitgestellt wird, derart auszuwerten, dass die erfasste elektromagnetische Strahlung nicht nur spektral analysiert werden kann, sondern mittels der selbstlernenden Auswerteeinheit trotz lediglich geringer Unterschiede im Spektrum eindeutig einem speziellen spezifischen Gewebetyp oder entsprechenden unterschiedlichen Zuständen zugeordnet werden kann, um zum Beispiel zu erkennen, ob das Gewebe entzündlich, nekrotisch, gutartig, bösartig und/oder dergleichen ist.

Die selbstlernende Auswerteeinheit wird initial anhand von Trainingsdatensätzen auf Basis von wenigstens einem Modell trainiert. Das Modell kann zum Beispiel durch ein oder mehrere neuronale Netze, Partial Least Squares, kernel-basierte Verfahren und/oder andere Verfahren des maschinellen Lernens gebildet sein. Die Auswerteeinheit umfasst vorzugsweise eine Hardwareschaltung, die insbesondere eine programmgesteuerte Rechnereinheit umfassen kann. Die Rechnereinheit wird mittels eines Rechnerprogramms derart gesteuert, dass sie die gewünschte Funktionalität bereitzustellen vermag.

Das Trainieren der Auswerteeinheit erfolgt mittels der Trainingsdatensätze, die ein jeweiliges Trainingssensorsignal mit einem jeweils zugeordneten Trainingsgewebetyp umfassen. Vorzugsweise wird eine große Anzahl von Trainingsdatensätzen genutzt, um die selbstlernende Auswerteeinheit zu trainieren. Die Trainingsdatensätze können anhand von Gewebeproben invivo oder auch in-vitro gewonnen werden. Die Trainingsdatensätze umfassen erfindungsgemäß Daten von einer Mehrzahl von unterschiedlichen Individuen und vorzugsweise möglichst vielen unterschiedlichen Gewebetypen. Die Trainingsdatensätze sind in einer geeigneten Datenbank gespeichert, die mit der Auswerteeinheit in Kommunikationsverbindung steht. Natürlich kann die Datenbank zumindest teilweise auch von der Auswerteeinheit umfasst sein. Die Hardwareschaltung, insbesondere die programmgesteuerte Rechnereinheit, kann dann anhand eines geeigneten Algorithmus eine zuverlässige Zuordnung eines Sensorsignals zu einem Gewebetyp realisieren.

Um den Operateur während des chirurgischen Eingriffs beziehungsweise der Operation unterstützen zu können, kann eine rechnerische und zeitnahe Auswertung der von dem Strahlungssensor bereitgestellten Sensorsignale durchgeführt werden, um jeweilige spezifische Gewebetypen ermitteln zu können. Vorzugsweise kann dies zum Beispiel während einer Tumorresektion durchgeführt werden, um die Operation möglichst effizient und mit möglichst wenig Nebenwirkungen für das Individuum beziehungsweise den Patienten zu realisieren.

Das Gewebe kann ein biologisches Gewebe des Individuums sein, das zum Beispiel ein Tier oder auch ein Mensch sein kann. Das Gewebe kann unterschiedliche Konsistenz aufweisen, beispielsweise elastisch, fest, fluid oder dergleichen. Ein fluides Gewebe kann zum Beispiel Blut, Liquor und/oder dergleichen sein. Das Gewebe kann ein organisches Gewebe sein, beispielsweise Lebergewebe, Darmgewebe, Muskelgewebe oder dergleichen, oder es kann auch ein Bindegewebe, ein Fettgewebe und/oder dergleichen sein.

Die Gewebeprobe gibt elektromagnetische Strahlung ab. Die abgegebene elektromagnetische Strahlung kann von der Gewebeprobe als solcher erzeugt und abgegeben werden. Es kann aber auch vorgesehen sein, dass die Gewebeprobe zur Abgabe der elektromagnetischen Strahlung angeregt wird. Das Anregen kann zum Beispiel mittels einer elektromagnetischen Anregungsstrahlung, beispielsweise Licht, insbesondere Infrarotlicht, ultraviolettes Licht, Kombinationen hiervon und/oder dergleichen sein. Die Gewebeprobe kann die Anregungsstrahlung reflektieren, transmittieren und/oder dergleichen. Natürlich kann die Gewebeprobe das Anregungslicht auch zumindest teilweise verändern beziehungsweise konvertieren, zum Beispiel hinsichtlich seiner spektralen Eigenschaften, einer spezifischen Strahlungsstärke und/oder dergleichen.

Der Strahlungssensor ist für das Erfassen der elektromagnetischen Strahlung geeignet ausgebildet. Soll mit dem Strahlungssensor zum Beispiel Infrarotstrahlung beziehungsweise Infrarotlicht erfasst werden, ist er vorzugsweise als Infrarotsensor ausgebildet. Soll er zum Beispiel ultraviolettes Licht beziehungsweise ultraviolette Strahlung erfassen können, ist er vorzugsweise als Ultraviolettsensor ausgebildet. Natürlich können auch andere Bereiche der elektromagnetischen Strahlung genutzt werden. Darüber hinaus können natürlich auch nahezu beliebige Kombinationen hiervon vorgesehen sein.

Der Strahlungssensor stellt das Sensorsignal bereit, welches abhängig von der erfassten elektromagnetischen Strahlung ist. Der Strahlungssensor ist zur Erfassung einer vorgegebenen spektralen Breite der zu erfassenden elektromagnetischen Strahlung ausgebildet. Entsprechend wird das Sensorsignal vom Strahlungssensor bereitgestellt. Es enthält also Informationen über die spektrale Verteilung der erfassten elektromagnetischen Strahlung.

Das Sensorsignal wird mittels der Auswerteeinheit ausgewertet. Vorzugsweise ist diesbezüglich vorgesehen, dass das Sensorsignal einer spektralen Analyse unterzogen wird. Die spektrale Analyse kann zum Beispiel mittels einer FourierTransformation oder dergleichen durchgeführt werden. Je nach Signalart kann auch eine Laplace-transformation, eine Z-Transformation und/oder dergleichen vorgesehen sein. Soll die Auswertung digital erfolgen, insbesondere unter Nutzung einer Rechnereinheit, kann das Sensorsignal zunächst in geeigneter Weise digitalisiert werden, um sodann eine entsprechende spektrale Analyse ebenfalls digital durchzuführen. Anhand des auf diese Weise ermittelten Spektrums beziehungsweise einer spektralen Verteilung der erfassten elektromagnetischen Strahlung kann dann unter Nutzung der Datenbank der Gewebetyp ermittelt werden.

Zu diesem Zweck wird die zuvor bereits mittels der Trainingsdatensätze initial trainierte selbstlernende Auswerteeinheit genutzt. Da - wie zuvor bereits erläutert - spektrale Unterschiede von unterschiedlichen Gewebetypen sehr gering sein können, kann eine unmittelbare Zuordnung anhand einer konventionellen Datenbank mit dort gespeicherten Datensätzen in der Regel nicht sinnvoll realisiert werden. Durch die selbstlernende Funktionalität der Auswerteeinheit kann die Funktionalität der Auswerteeinheit diesbezüglich erheblich sensibilisiert werden, wodurch die Zuordnung eines jeweiligen Gewebetyps zu einem jeweiligen erfassten Sensorsignal erheblich verbessert beziehungsweise zuverlässiger erfolgen kann. Ist die selbstlernende Auswerteeinheit nämlich hinreichend gut trainiert, kann eine hohe Zuverlässigkeit beim Ermitteln des Gewebetyps realisiert werden.

Da die Erfindung es ferner erlaubt, die Gewebeprobe zeitnah hinsichtlich des Gewebetyps zu untersuchen, kann mit der Erfindung eine zuverlässige Unterstützung des Operateurs auch während der Operation am Individuum erreicht werden. Es bedarf also nicht mehr des Übersendens des entnommenen Gewebes zu einer separaten histologischen Untersuchungsstation, die dann eine aufwändige Untersuchung durchführt und eine entsprechende Information über den ermittelten Gewebetyp an den Operateur übermittelt, währenddessen im Stand der Technik das Individuum weiterhin narkotisiert zu sein hat. Die hiermit verbundenen Risiken können durch Nutzung der Erfindung somit weitgehend vermieden werden.

Die Auswerteeinheit wird nach einem Ermitteln des Gewebetyps der Gewebeprobe unter Berücksichtigung von einem diesem Auswerten zugeordneten Datensatz erneut trainiert. Die Auswerteeinheit kann also während des bestimmungsgemäßen Betriebs hinsichtlich ihrer Funktionalität weiter verbessert werden.

Vorzugsweise kann vorgesehen sein, dass nach jedem Ermitteln eines Gewebetyps der zugrundeliegende Datensatz dazu genutzt wird, die Auswerteeinheit erneut zu trainieren. Natürlich kann auch vorgesehen sein, dass das erneute Trainieren zu vorgebbaren Zeitpunkten oder auch in vorbestimmten Wartungsintervallen erfolgt. Zu diesem Zweck können die entsprechenden Datensätze bis zum Durchführen des Trainings separat gespeichert werden. Sie können auch neue Trainingsdatensätze bilden. Das erneute Trainieren wird während der bestimmungsgemäßen Nutzung, beispielsweise während einer Operation, realisiert. Dadurch ist es möglich, die selbstlernende Auswerteeinheit an spezifische Situationen, die während einer Operation am Individuum auftreten können, kurzfristig anpassen zu können. Die Funktionalität der selbstlernenden Auswerteeinheit kann dadurch weiter verbessert werden, insbesondere auch die Unterstützung des Operateurs durch die Erfindung.

Vorzugsweise enthält die Gewebeprobe einen Markerstoff, der die durch die Gewebeprobe abgegebene elektromagnetische Strahlung beeinflusst. Der Markerstoff kann zum Beispiel das Gewebe zur Abgabe der elektromagnetischen Strahlung anregen. Es kann sogar vorgesehen sein, dass der Markerstoff selbst die elektromagnetische Strahlung abgibt. Der Markerstoff kann zum Beispiel ein Stoff sein, der sich gewebespezifisch verhält, das heißt, in einem spezifisch vorgegebenen Gewebe bevorzugt anreichert. Es kann aber auch vorgesehen sein, dass der Markerstoff während der Operation hinzugefügt wird, und sich lediglich an einem spezifisch vorgegebenen Gewebe anhaftet. Vorzugsweise hat der Markerstoff spezifische Eigenschaften bezüglich der Abgabe beziehungsweise der Beeinflussung von elektromagnetischer Strahlung, sodass er die Detektion eines spezifischen Gewebetyps erleichtert. Der Markerstoff kann zum Beispiel selbst elektromagnetische Strahlung abgeben, zum Beispiel Infrarotlicht oder dergleichen. Es kann auch vorgesehen sein, dass der Markerstoff durch eine Anregungsstrahlung, insbesondere Anregungslicht, zur Abgabe von elektromagnetischer Strahlung, insbesondere Licht, angeregt werden kann. Beispielsweise kann vorgesehen sein, dass der Markerstoff eine Konversion der Anregungsstrahlung in eine vorgegebene abgegebene elektromagnetische Strahlung vornimmt. Es kann auch vorgesehen sein, dass der Markerstoff das Abgeben von elektromagnetischer Strahlung durch die Gewebeprobe unterstützt oder verstärkt.

Es wird ferner vorgeschlagen, dass die Trainingsdatensätze den Trainingssensorsignalen zugeordnete spezifizierbare Trainingsgewebetypen von Tieren und/oder Menschen umfassen. Hierdurch kann erreicht werden, dass durch Trainingsdatensätze von einer möglichst großen Vielfalt von Individuen die selbstlernende Auswerteeinheit besonders gut trainiert werden kann. Dabei kann berücksichtigt werden, dass zum Trainieren der selbstlernenden Auswerteeinheit häufig eine große Vielzahl von Trainingsdatensätzen erforderlich ist, die von einem einzigen Individuum in der Regel nicht erreicht werden kann. Besonders vorteilhaft erweist es sich, wenn die spezifizierbaren Trainingsgewebetypen gesundes Gewebe und/oder Tumorgewebe umfassen. Dadurch kann erreicht werden, dass die selbstlernende Auswerteeinheit besonders im Bereich der Unterscheidung von gesundem Gewebe und Tumorgewebe trainiert werden kann.

Weiterhin wird vorgeschlagen, dass erfindungsgemäß vor dem Ermitteln des Gewebetyps der Gewebeprobe die Auswerteeinheit mittels wenigstens eines weiteren bekannten Datensatzes des gleichen Individuums trainiert wird. Beispielsweise kann dieser Datensatz erzeugt werden, in dem vor einer Operation vom Individuum eine entsprechende Gewebeprobe bereitgestellt wird und mittels des erfindungsgemäßen Verfahrens untersucht wird, zum Beispiel im Rahmen einer Biopsie oder dergleichen. Es können natürlich auch entsprechend mehrere Gewebeproben des Individuums vor der geplanten Operation entsprechend untersucht werden, um die Funktionalität der selbstlernenden Auswerteeinheit besonders gut an das Individuum anpassen zu können. Dies kann die bestimmungsgemäße Unterstützung des Operators während des chirurgischen Eingriffs beziehungsweise der Operation verbessern. Hierdurch kann erreicht werden, dass in-vivo-Gewebeproben als auch ex-vivo-Geweberoben genutzt werden können. So können auch pathologisch untersuchte ex-vivo-Gewebeproben von unterschiedlichen Individuen zum Trainieren genutzt werden. Besonders vorteilhaft erweist sich dies für die Funktionalität sowie auch für eine ärztliche Akzeptanz, insbesondere weil Ergebnisse nicht von einem Tiermodell auf den Menschen übertragen zu werden brauchen. Weiterhin kann es vorteilhaft sein, wenn ein "negatives Lernen" ermöglicht ist, wenn das Lernen mit Gewebeproben erfolgt, von denen bekannt ist, dass es sich um auffälliges Gewebe, insbesondere Tumorgewebe, handelt und dann in der Anwendung weiter analysiert wird, ob die aktuelle Gewebeprobe noch zu der gleichen Klasse gehört oder gegebenenfalls einer anderen Klasse zuzuordnen ist.

Weiterhin wird vorgeschlagen, dass die Auswerteeinheit wenigstens zwei voneinander verschiedene Modelle nutzt, wobei vor dem Auswerten des Sensorsignals eine Validierung der Modelle erfolgt, indem eine Eignung der jeweiligen Modelle für das spezifische, die Gewebeprobe bereitstellende Individuum geprüft wird. Es besteht also durch diese Weiterbildung die Möglichkeit, eines oder mehrere von vorhandenen Modellen für die Auswertung durch die selbstlernende Auswerteeinheit bevorzugt zu nutzen. Beispielsweise kann die Gewebeprobe mittels der verfügbaren Modelle durch die selbstlernende Auswerteeinheit untersucht werden, indem abhängig von einem jeweiligen Modell der entsprechende Gewebetyp ermittelt wird. Das Ergebnis kann durch eine dritte Person, insbesondere den Operateur, überprüft werden. Anhand dieser Überprüfung kann der Operateur dann vorgeben, welches der jeweiligen Modelle für weitere Untersuchungen beziehungsweise die Ermittlung von Gewebetypen herangezogen werden soll. Je nach Individuum kann dies variieren.

Vorzugsweise nutzt die Auswerteeinheit zum Auswerten wenigstens eine Gewichtungsfunktion. Mit der Gewichtungsfunktion kann die Relevanz eines jeweiligen Auswerteergebnisses berücksichtigt werden. Die Gewichtungsfunktion kann entweder durch den Operateur vorgegeben werden oder auch beim Trainieren entsprechend eingestellt werden.

Besonders vorteilhaft erweist es sich, wenn einem jeweiligen Modell zum Auswerten jeweils eine eigene Gewichtungsfunktion zugeordnet wird. Diese Option richtet sich insbesondere darauf, dass die Auswerteeinheit nicht nur ein einziges Modell zum Auswerten nutzt, sondern zwei oder mehrere Modelle. Je nach Relevanz der erzielten Ergebnisse kann die Gewichtungsfunktion einem jeweiligen Modell eine individuelle Gewichtung zuordnen. Dies ermöglicht es, dass Ermitteln des Gewebetyps weiter zu verbessern.

Besonders vorteilhaft erweist es sich, wenn die Gewebeprobe mit einer vorgebbaren elektromagnetischen Strahlung beaufschlagt wird. Die vorgebbare elektromagnetische Strahlung kann beispielsweise eine Infrarotstrahlung, eine UV-Strahlung, sichtbares Licht, Kombinationen hiervon und/oder dergleichen sein. Es kann natürlich auch vorgesehen sein, dass die Gewebeprobe lediglich mit einer monochromatischen elektromagnetischen Strahlung beaufschlagt wird, und der Strahlungssensor nicht nur sensitiv für die monochromatische elektromagnetische Strahlung ist, sondern auch weitere Strahlungsbereiche erfasst. Dies erlaubt es, spezifische spektrale Veränderungen, die durch Wechselwirkungen der Gewebeprobe mit der monochromatischen elektromagnetischen Strahlung erfolgen können, zu erfassen. Beispielsweise können so Konversionen erfasst werden, bei denen die monochromatische elektromagnetische Strahlung durch die Gewebeprobe spektral zumindest teilweise verändert werden. Insgesamt kann hierdurch die Funktionalität der Erfindung weiter verbessert werden.

Die Auswerteeinheit kann durch eine elektronische Schaltung, eine Rechnereinheit, Kombinationen hiervon und/oder dergleichen gebildet sein. Die Rechnereinheit kann derart eingerichtet sein, dass sie das Verfahren gemäß der Erfindung zumindest teilweise ausführen kann, zu welchem Zweck sie mittels des Rechnerprogramms entsprechend gesteuert wird. Sowohl die Rechnereinheit als auch die elektronische Schaltung können durch einen oder mehrere Halbleiter-Chips gebildet sein. Darüber hinaus kann die Auswerteeinheit auch durch diskrete elektronische Bauelemente sowie Kombinationen mit Halbleiter-Chips gebildet sein. Insbesondere kann die Auswerteeinheit eine Speichereinheit umfassen, in der zumindest teilweise die Trainingsdatensätze hinterlegt sein können. Darüber hinaus kann in der Speichereinheit zumindest teilweise das Rechnerprogramm für die Rechnereinheit gespeichert sein.

Das vorgenannte Rechnerprogrammprodukt kann als rechnerlesbares Speichermedium ausgebildet sein. Darüber hinaus kann das Rechnerprogramm direkt in einen internen Speicher der Rechnereinheit ladbar sein. So ist es beispielsweise möglich, das Rechnerprogramm aus einem Netzwerk, insbesondere dem Internet, von einer Datenquelle, beispielsweise einem Server, herunterzuladen und in einem internen Speicher der Rechnereinheit zu laden, sodass die Rechnereinheit das Rechnerprogramm ausführen kann. Vorzugsweise umfasst das Rechnerprogrammprodukt ein rechnerlesbares Medium, auf welchem die Programmkodeabschnitte gespeichert sind. Ein solches rechnerlesbares Medium kann beispielsweise ein Speicherbaustein, beispielsweise ein PROM, eine Compact-Disk, ein USB-Stick oder dergleichen sein.

Weitere Vorteile und Merkmale ergeben sich durch die Beschreibung eines Ausführungsbeispiels anhand der beigefügten Figuren. In den Figuren bezeichnen gleiche Bezugszeichen gleiche Merkmale und Funktionen.

Es zeigen die Figuren:
- Fig. 1: eine schematische Blockdarstellung für einen Aufbau zur Durchführung des Verfahrens gemäß der Erfindung und
- Fig. 2: in einer schematischen Flussdiagrammdarstellung einen Ablauf eines Verfahrens in Zusammenhang mit der Erfindung.

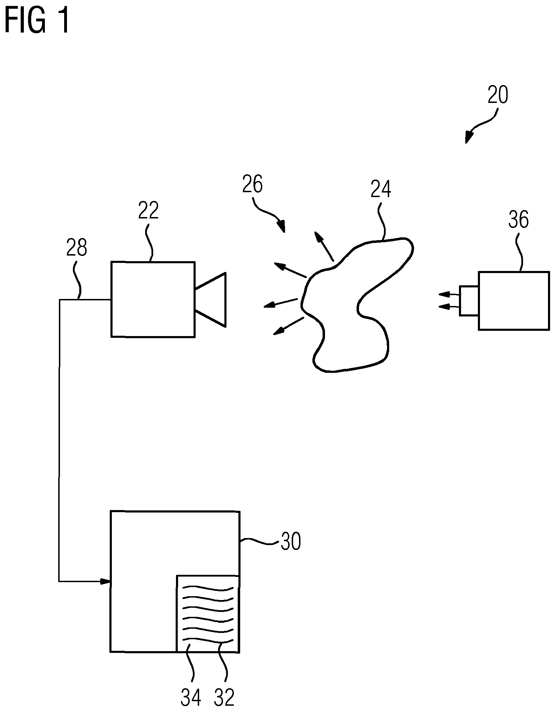

Fig. 1 zeigt in einer schematischen Blockdarstellung eine Vorrichtung 20 zum Ermitteln eines Gewebetyps eines Gewebes eines tierischen oder menschlichen Individuums. Die Vorrichtung 20 umfasst einen Strahlungssensor 22 zum Erfassen von durch eine Gewebeprobe 24 des Gewebes abgegebener elektromagnetischer Strahlung 26. Der Strahlungssensor 22 stellt abhängig von der erfassten elektromagnetischen Strahlung ein Sensorsignal 28 bereit. Vorliegend handelt es sich bei der elektromagnetischen Strahlung 26 um Infrarotstrahlung. Entsprechend ist der Strahlungssensor 22 als Infrarotsensor ausgebildet.

Die Vorrichtung 20 umfasst ferner einer Auswerteeinheit 30 zum Auswerten des Sensorsignals 28, welches vorliegend ein elektrisches Signal ist. Das Sensorsignal 28 ist ferner vorliegend ein analoges Signal.

Um den Gewebetyp der Gewebeprobe 24 zu ermitteln, ist die Auswerteeinheit 30 als selbstlernende Auswerteeinheit 30 ausgebildet. Ferner ist sie initial anhand von Trainingsdatensätzen 32, die in einer Datenbank 34 der Auswerteeinheit 30 gespeichert sind, auf Basis von wenigstens einem Modell trainiert worden. Das Modell ist vorliegend ein mehrschichtiges neuronales Netz, das auf einem Verfahren zum maschinellen Lernen basierend trainiert werden kann. Das Trainieren der Auswerteeinheit 30 ist mit solchen Trainingsdatensätzen 32 vorgesehen, die ein jeweiliges Trainingssensorsignal mit einem jeweils zugeordneten Trainingsgewebetyp umfassen.

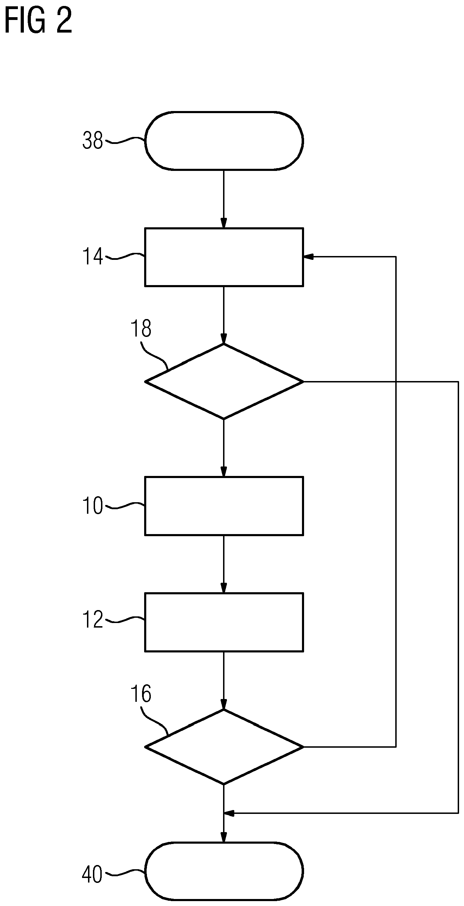

Fig. 2 zeigt einen schematischen Ablauf für ein Verfahren in Zusammenhang mit der Erfindung. Das Verfahren beginnt im Schritt 38. Im folgenden Schritt 14 wird die Auswerteeinheit 30 initial anhand von den Trainingsdatensätzen 32 auf Basis von wenigstens einem Modell trainiert.

Das Verfahren wird mit Schritt 18 fortgesetzt, bei dem geprüft wird, ob eine Gewebeprobe 24 vorliegt. Liegt keine Gewebeprobe vor beziehungsweise keine neue Gewebeprobe, so springt das Verfahren zum Schritt 40, der das Verfahren beendet.

Ist hingegen eine Gewebeprobe 24 beziehungsweise eine neue Gewebeprobe 24 vorhanden, wird im Schritt 10 die durch die Gewebeprobe 24 abgegebene elektromagnetische Strahlung 26, vorliegend Infrarotlicht, mittels des Strahlungssensors 22 erfasst. Der Strahlungssensor 22 stellt abhängig von der erfassten elektromagnetischen Strahlung 26 das Sensorsignal 28 bereit.

Das Verfahren 20 wird dann mit dem Schritt 12 fortgeführt, bei dem mittels der Auswerteeinheit 30 das Sensorsignal 28 ausgewertet wird, um den Gewebetyp der Gewebeprobe 24 zu ermitteln. Zu diesem Zweck wird das Sensorsignal 28, welches vorliegend ein analoges Signal ist, zunächst digitalisiert und einer digitalen Signalverarbeitung durch die Auswerteeinheit 30 zugeführt. Die Auswerteeinheit 30 umfasst hierfür eine nicht weiter dargestellte Rechnereinheit, die mittels eines geeignet ausgebildeten Rechnerprogramms gesteuert wird, um die erforderliche Funktionalität gemäß der Erfindung realisieren zu können. Am Ende des Schritts 12 wird der ermittelte Gewebetyp ausgegeben. Die Ausgabe kann entweder akustisch, visuell oder auch haptisch erfolgen, beispielsweise mittels einer Lautsprecherwiedergabe, einer Darstellung auf einem Monitor, durch ein Lichtsignal, durch ein Vibrieren an einem Bedienteil und/oder dergleichen.

Das Verfahren wird mit einer Verzweigung 16 weitergeführt, bei der geprüft wird, ob das Ermitteln des Gewebetyps mit einer hinreichend hohen Zuverlässigkeit erfolgte. Zu diesem Zweck kann durch einen nicht dargestellten Operateur ein vorgegebener Zuverlässigkeitswert vorgegeben werden. Erfolgt das Ermitteln des Gewebetyps mit einer entsprechend hohen Zuverlässigkeit, verzweigt sich das Verfahren zum Schritt 14, wo unter Berücksichtigung des durch das Ermitteln des Gewebetyps neu ermittelten Datensatzes die Auswerteeinheit 30 erneut trainiert wird. Wurde die vorgegebene Zuverlässigkeit nicht erreicht, wird das Verfahren mit dem Schritt 40 fortgeführt und beendet.

Die selbstlernende Auswerteeinheit 30 benötigt vorzugsweise für den Schritt 14 des Trainierens eine Vielzahl von Trainingsdatensätzen 32. Häufig ist es jedoch schwierig, für Zulassung einer solchen Methode ausreichend viele Trainingsdatensätze 32 von humanen Geweben zu erhalten, um eine entsprechend belastbare Datenbank 34 bereitstellen zu können, damit die Auswerteeinheit 30 zuverlässig trainiert werden kann.

Um die vorgenannte Problematik zu lösen, können die folgenden ergänzenden Schritte vorgesehen sein.

Ein erster derartiger Schritt kann vor einer ersten Nutzung der Auswerteeinheit 30 durchgeführt werden, das heißt, insbesondere präoperativ. Die Auswerteeinheit 30 wird hier zunächst anhand von Tiermodellen trainiert.

Zu diesem Zweck kann vorgesehen sein, dass Infrarotspektren an einem Tiermodell ermittelt werden. Hieraus können die Trainingsdatensätze 32 ermittelt werden, die in der Datenbank 34 gespeichert werden können. Die hierbei untersuchten Gewebeproben 24 enthalten vorzugsweise normales beziehungsweise gesundes Gewebe, zum Beispiel vom Gehirn, und vorzugsweise auch Tumorgewebe beziehungsweise auffälliges Gewebe, vorzugsweise desselben Gewebetyps wie bezüglich des gesunden Gewebes, zum Beispiel von einem Gliom, Glioblastom und/oder dergleichen.

Im Folgenden können die von den Gewebeproben der Tiere aufgenommenen Spektren beziehungsweise Sensorsignale 28, vorzugsweise derselben Spezies, beispielsweise einer Ratte, Sprague Dawley oder dergleichen, analysiert und validiert werden. Insbesondere kann hier eine histologische Untersuchung durchgeführt werden.

Die Datenbank 34 kann durch die hierbei gewonnenen Trainingsdatensätze 32 von normalen beziehungsweise gesunden Gewebe und/oder auffälligem Gewebe, insbesondere Tumorgewebe, vorzugsweise desselben Gewebetyps erweitert werden.

Weiterhin können verschiedene Gewebetypen einer anderen Spezies, zum Beispiel Fisher Rats oder dergleichen, berücksichtigt werden. Besonders vorteilhaft erweist es sich, wenn für die Erstellung der Trainingsdatensätze 32 normierte Tiere herangezogen werden können, die unter normierten Bedingungen gehalten werden können.

Es erfolgt eine weitere Analyse und Validierung der für die unterschiedlichen Spezies von Tieren gewonnenen ergänzenden Trainingsdatensätze 32.

Im Folgenden wird die Datenbank 34 durch die hierbei gewonnenen Trainingsdatensätze 32 ergänzt. Als Tiere kommen natürlich auch Haustiere wie Hund, Schwein, Kuh aber auch Katze, Maus und/oder dergleichen infrage.

Es erfolgt eine erneute Validierung der Trainingsdatensätze 32, woraufhin dann eine entsprechende Datenbank 34 unter Berücksichtigung der hierbei gewonnenen Trainingsdatensätze 32 erstellt wird.

Eine weitere Erweiterung der Datenbank 34 kann mittels Trainingsdatensätzen 32 erreicht werden, die unter Nutzung von Gewebeproben 24 von humanem Gewebe, insbesondere präoperativ, bereitgestellt werden.

Hierzu können entsprechende Trainingsdatensätze 32 unter Nutzung von Gewebeproben 24 von humanem Gewebe bereitgestellt werden, beispielsweise normales beziehungsweise gesundes Gewebe, zum Beispiel vom Gehirn oder dergleichen, sowie auch auffälliges Gewebe, insbesondere Tumorgewebe, vorzugsweise desselben Gewebetyps, zum Beispiel ein Gliom, ein Glioblastom und/oder dergleichen sowie auch von unterschiedlichen Gewebetypen.

Es erfolgt eine Analyse und Validierung der entsprechend hierbei gewonnenen Trainingsdatensätze 32, vorzugsweise auch von dem gleichen Individuum beziehungsweise Patienten aufgenommen. Daraufhin erfolgt ein Abgleich mit der Datenbank 34.

Es erfolgt dann eine weitergehende Analyse und Validierung der Trainingsdatensätze 32, die von unterschiedlichen Individuen beziehungsweise Patienten aufgenommen wurden. Wiederum erfolgt ein Abgleich mit der Datenbank 34.

Schließlich wird die Datenbank 34 um die hierdurch gewonnenen Trainingsdatensätze 32 erweitert.

Mit dem vorgenannten Vorgehen kann somit eine initiale Datenbank 34 bereitgestellt werden, die eine Vielzahl von Trainingsdatensätzen 32 bereitzustellen vermag, damit die Auswerteeinheit 30 initial trainiert werden kann.

Die Anpassung beziehungsweise das Training der Auswerteeinheit 30 braucht aber mit dem vorgenannten Vorgehen nicht abgeschlossen zu sein. Die Erfindung erlaubt es nämlich, dass eine Anpassung entsprechender Auswertemodelle der Auswerteeinheit 30 auch während der Nutzung beziehungsweise der Verfahrensführung gemäß der Erfindung realisiert werden kann.

So kann zum Beispiel vorgesehen sein, dass verschiedene Gewebeproben von Nicht-Tumorgewebe erfasst werden, die als interne Referenzen dienen können. Zu diesem Zweck kann der Operateur vorzugsweise den Gewebetyp vorgeben, sodass ein jeweiliger Trainingsdatensatz 32 entsprechend bereitgestellt werden kann.

Die Aufnahme der hierbei gewonnenen Trainingsdatensätze 32 kann als ergänzende interne Referenz dienen, und es wird ein weiterer Abgleich mit der Datenbank 34 durchgeführt.

Sodann kann mit dem chirurgischen Eingriff beziehungsweise der Operation begonnen werden, beispielsweise eine Entfernung eines Tumors, wobei die gemäß der Erfindung ermittelte Gewebetypisierung herangezogen werden kann, um den chirurgischen Eingriff beziehungsweise die Operation durch den Operateur zu steuern.

Die hierbei dem ermittelten Kontrolldatensätze in Bezug auf bekannte Gewebetypen können dabei auf zwei Arten eingesetzt werden:
Einerseits kann eine Validierung erfolgen, indem zunächst eine Validierung von Modellen vor einer Operation beziehungsweise einem chirurgischen Eingriff durchgeführt wird. Dabei wird die Eignung eines jeweiligen der Modelle für den spezifischen Patienten beziehungsweise das spezifische Individuum geprüft. Modelle, deren Vorhersage beziehungsweise Klassifizierung über einen vorgebbaren Wert vom durch den Operateur vorgegebenen Zielwert, hier zum Beispiel Tumor beziehungsweise Nicht-Tumor, abweicht, können für die Berücksichtigung während der Operation ausgeschlossen werden.

Alternativ oder ergänzend kann auch eine Rekalibrierung und/oder ein Neutraining vorgesehen sein. Hier sind mehrere Vorgehensweisen möglich. Im einfachsten Fall können Kontrolldatensätze zur Anpassung einer nichtlinearen Schwellwert-oder Ausgabefunktion verwendet werden, die eine Modellausgabe in eine Klassifikationsentscheidung abbildet. Beispielsweise kann eine Modellvorhersage von etwa 10 % Tumorwahrscheinlichkeit und eine Vorgabe des Operators von 0 % dazu führen, dass ein Ausgabewert des Modells nach unten korrigiert wird. In einer anderen Realisierung kann dagegen eine Gewichtungsfunktion gelernt werden, die mehrere Modelle geeignet kombiniert. Dabei kann jedes Modell ein eigenes Gewicht beziehungsweise sogar eine eigene Gewichtungsfunktion zugeordnet bekommen, die deren Eignung für den spezifischen chirurgischen Eingriff beziehungsweise die Operation beziehungsweise das spezifische Individuum darstellt. Die Gewichte können anhand von Kontrolldatensätzen so trainiert werden, dass ein kombinierter Gesamtwert maximal mit der Vorgabe des Operators übereinstimmt.

Da eine Anzahl von Trainingsdatensätzen 32, die zum Beispiel während einer Operation ermittelt werden können, in der Regel relativ gering sein wird, und daher allgemein nicht für das Trainieren der Auswerteeinheit 30 ausreichen dürfte, kann ergänzend auch noch folgendes vorgesehen werden:
Der Operateur gibt ein jeweils zu ermittelnden Gewebetyp in Verbindung mit einem zu klassifizierendes Testspektrum beziehungsweise Testsensorsignal als einen Testdatensatz vor. Mittels eines geeigneten Algorithmus kann dann aus der Gesamtmenge aller Trainingsdatensätze 32 der Datenbank 34 eine Anzahl von Trainingsdatensätzen 32 ermittelt werden, die vorzugsweise am ähnlichsten zum vorgegebenen Testdatensatz sind. Zu diesem Zweck kann ein geeignetes Ähnlichkeitsmaß angegeben werden. Eine Anzahl solcher Trainingsdatensätze kann zum Beispiel 500 sein.

Zu den auf diese Weise ermittelten Trainingsdatensätzen 32 können ergänzend die während einer Operation ermittelten ergänzenden Trainingsdatensätze 32 hinzugefügt werden. Der hierdurch gebildete Teil der Datenbank 34 kann dann zum Trainieren der Auswerteeinheit 30 herangezogen werden, und zwar vorzugsweise unter Benutzung eines lokalen Modells für die Klassifikation beziehungsweise die Ermittlung des Gewebetyps. Beispielsweise kann können hierfür Partial Least Squares herangezogen werden. Das auf diese Weise gewonnene Modell kann dann validiert und für die Klassifikation des vorgegebenen Testdatensatzes genutzt werden.

Mittels aktuell verfügbarer Hardware, insbesondere auch Rechnereinheiten, sollte es möglich sein, die erfindungsgemäße Verfahrensführung mit einer Geschwindigkeit auszuführen, dass die Nutzung auch während der Operation für den Operateur zur Verfügung steht.

Darüber hinaus kann natürlich auch vorgesehen sein, dass die während der Operation ermittelten Trainingsdatensätze gespeichert und mit einem geeigneten Lernverfahren auch unabhängig von einem Zielwert zum Training eines Modells der Auswerteeinheit 30 herangezogen werden können. Hierzu können zum Beispiel Methoden des halbüberwachten Lernens (Semi Supervised Learning) sowie auch Verfahren aus dem Bereich Deep Learning (zum Beispiel Restricted Boltzmann Machines) oder dergleichen genutzt werden.

Das vorgenannte Ausführungsbeispiel dient ausschließlich der Erläuterung der Erfindung und soll diese nicht beschränken. Insbesondere ist das beschriebene Verfahren der Erfindung nicht auf eine Klassifikation von Gewebetypen wie Tumor oder Nicht-Tumor beschränkt. Es kann auch auf eine Vorhersage von kontinuierlichen Größen beziehungsweise eine Regression, beispielsweise einen Blutalkoholgehalt oder dergleichen, angewendet werden.

## Patentansprüche

1. Verfahren zum Ermitteln eines Gewebetyps eines entnommenen Gewebes eines tierischen oder menschlichen Individuums, bei dem:
- durch eine Gewebeprobe (24) des Gewebes abgegebene elektromagnetische Strahlung (26) mittels eines Strahlungssensors (22) erfasst (10) wird, wobei der Strahlungssensor (22) abhängig von der erfassten elektromagnetischen Strahlung ein Sensorsignal (28) bereitstellt, und
- das Sensorsignal (28) mittels einer Auswerteeinheit (30) ausgewertet (12) wird, um den Gewebetyp zu ermitteln und auszugeben, wobei die Auswerteeinheit (30) eine selbstlernende Auswerteeinheit (30) ist, die initial anhand von Trainingsdatensätzen (32) auf Basis von wenigstens einem Modell trainiert (14) wird, das auf einem Verfahren zum maschinellen Lernen basiert, wobei das Trainieren der Auswerteeinheit mittels solcher Trainingsdatensätze (32) erfolgt, die ein jeweiliges Trainingssensorsignal mit einem jeweils zugeordneten Trainingsgewebetyp umfassen, wobei die Trainingsdatensätze (32) die Daten von einer Mehrzahl von unterschiedlichen Individuen umfassen, die in einer geeigneten Datenbank gespeichert sind, **dadurch gekennzeichnet, dass**
die Auswerteeinheit (30) vor einem Ermitteln des Gewebetyps der Gewebeprobe (24) des tierischen oder menschlichen Individuums mittels wenigstens eines weiteren bekannten Datensatzes des gleichen Individuums zur Anpassung an das tierische oder menschliche Individuum trainiert (16) wird.

2. Verfahren nach Anspruch 1, wobei die Gewebeprobe einen Markerstoff enthält, der die durch die Gewebeprobe abgegebene elektromagnetische Strahlung beeinflusst.

3. Verfahren nach Anspruch 1 oder 2, wobei die Trainingsdatensätze (32) den Trainingssensorsignalen zugeordnete spezifizierbare Trainingsgewebetypen von Tieren und/oder Menschen umfassen.

4. Verfahren nach Anspruch 3, wobei die spezifizierbaren Trainingsgewebetypen gesundes Gewebe und/oder Tumorgewebe umfassen.

5. Verfahren nach Anspruch 4, wobei das Trainieren nur dann erfolgt, wenn bei dem Auswerten ein vorgegebener Zielwert erreicht wird.

6. Verfahren nach einem der vorhergehenden Ansprüche, wobei die Auswerteeinheit (30) wenigstens zwei voneinander verschiedene Modelle nutzt, wobei vor dem Auswerten des Sensorsignals (28) eine Validierung der Modelle erfolgt, indem eine Eignung der jeweiligen Modelle für das spezifische, die Gewebeprobe (24) bereitstellende Individuum geprüft wird.

7. Verfahren nach einem der vorhergehenden Ansprüche, wobei die Auswerteeinheit (30) zum Auswerten wenigstens eine Gewichtungsfunktion nutzt.

8. Verfahren nach Anspruch 7, wobei einem jeweiligen Modell zum Auswerten jeweils eine eigene Gewichtungsfunktion zugeordnet wird.

9. Verfahren nach einem der vorhergehenden Ansprüche, wobei die Gewebeprobe (24) mit einer vorgebbaren elektromagnetischen Strahlung beaufschlagt wird.

## Claims

1. Method for determining a tissue type of a tissue taken from an animal or human individual, in which method:
- a radiation sensor (22) detects (10) electromagnetic radiation (26) emitted by a tissue sample (24) of the tissue, said radiation sensor (22) providing a sensor signal (28) according to the detected electromagnetic radiation, and
- an analysis unit (30) analyses (12) the sensor signal (28) in order to determine and output the tissue type, wherein the analysis unit (30) is a self-learning analysis unit (30), which is initially trained (14) using training datasets (32) on the basis of at least one model based on a machine learning method, the training of said analysis unit being performed using said training datasets (32), which each comprise a training sensor signal with an associated training tissue type in each case, wherein the training datasets (32) comprise the data from a plurality of different individuals which are stored in a database, **characterised in that** before determining the tissue type of the tissue sample (24) of the animal or human individual by means of at least one further known dataset of the same individual, the analysis unit (30) is trained for adaptation to the animal or human individual.

2. Method according to claim 1, wherein the tissue sample contains a marker agent, which influences the electromagnetic radiation emitted by the tissue sample.

3. Method according to claim 1 or claim 2, wherein the training datasets (32) comprise specifiable training tissue types of animals and/or humans, which training tissue types are associated with the training sensor signals.

4. Method according to claim 3, wherein the specifiable training tissue types comprise healthy tissue and/or tumour tissue.

5. Method according to claim 4, wherein the training is performed only when a specified target value is reached in the analysis.

6. Method according to one of the preceding claims, wherein the analysis unit (30) uses at least two different models, wherein before the sensor signal (28) is analysed, the models are validated by checking a suitability of each of the models to the specific individual providing the tissue sample (24).

7. Method according to one of the preceding claims, wherein the analysis unit (30) uses at least one weighting function for the analysis.

8. Method according to claim 7, wherein each model for the analysis is assigned a dedicated weighting function.

9. Method according to one of the preceding claims, wherein the tissue sample (24) is exposed to definable electromagnetic radiation.

## Revendications

1. Procédé de détermination d'un type de tissu prélevé d'un individu animal ou humain, dans lequel :
- on détecte (10), au moyen d'un capteur (22) rayonnement, du rayonnement (26) électromagnétique donné par un échantillon (24) du tissu, dans lequel le capteur (22) de rayonnement donne un signal (28) de capteur en fonction du rayonnement électromagnétique détecté, et
- on analyse (12) le signal (28) de capteur, au moyen d'une unité (30) d'analyse, pour déterminer et sortir le type de tissu, dans lequel l'unité (30) d'analyse est une unité (30) d'analyse à autoapprentissage, qui subit initialement un apprentissage (14) à l'aide d'ensembles (32) de données d'apprentissage, sur la base d'au moins un modèle, qui repose sur un procédé d'apprentissage par machine, dans lequel l'apprentissage de l'unité d'analyse s'effectue au moyen d'ensembles (32) de données d'apprentissage, qui comprennent un signal de capteur d'apprentissage ayant un type de tissu d'apprentissage associé respectivement, dans lequel les ensembles (32) de données d'apprentissage comprennent les données d'une pluralité d'individus différents, qui sont mises en mémoire dans une base de données appropriées, **caractérisé en ce que** l'on fait , pour l'adaptation à l'individu animal ou humain, subir à l'unité (30) d'apprentissage, avant une détermination du type de tissu de l'échantillon (24) de tissu de l'individu animal ou humain, un apprentissage (16) au moyen d'au moins un autre ensemble de données connu du même individu.

2. Procédé suivant la revendication 1, dans lequel l'échantillon de tissu contient un marqueur, qui influence le rayonnement électromagnétique donné par l'échantillon de tissu.

3. Procédé suivant la revendication 1 ou 2, dans lequel les ensembles (32) de données d'apprentissage comprennent des types de tissu d'apprentissage associés aux signaux de capteur d'apprentissage et pouvant être spécifiés, d'animaux et/ou d'hommes.

4. Procédé suivant la revendication 3, **caractérisé en ce que** les types de tissu d'apprentissage pouvant être spécifiés comprennent des tissus sains et/ou des tissus de tumeurs.

5. Procédé suivant la revendication 4, dans lequel on n'effectue l'apprentissage que, si l'on atteint, à l'analyse, une valeur cible donnée à l'avance.

6. Procédé suivant l'une des revendications précédentes, dans lequel l'unité (30) d'analyse utilise au moins deux modèles différents l'un de l'autre, dans lequel, avant l'analyse du signal (28) de capteur, on effectue une validation des modèles, en contrôlant une appropriation des modèles respectifs à l'individu spécifique donnant l'échantillon (24) de tissu.

7. Procédé suivant l'une des revendications précédentes, dans lequel l'unité (30) d'analyse utilise, pour l'analyse, au moins une fonction de pondération.

8. Procédé suivant la revendication 7, dans lequel on affecte une fonction de pondération propre à chaque modèle pour l'analyse.

9. Procédé suivant l'une des revendications précédentes, dans lequel on soumet l'échantillon (24) de tissu à un rayonnement électromagnétique pouvant être donné à l'avance.
